# EUROPEAN PATENT SPECIFICATION

(11) **EP 3 349 779 B1**
(45) Date of publication and mention of the grant of the patent: **29.04.2020**
(21) Application number: 16766922.5
(22) Date of filing: 14.09.2016
(51) Int. Cl.: A61K 38/12, C07K 7/56, C07K 7/60, A61P 5/40

(54) **CYCLIC POLYPEPTIDE FOR THE TREATMENT OF PHA TYPE 1B**
ZYKLISCHES POLYPEPTID ZUR BEHANDLUNG VON PHA TYPE 1B
POLYPEPTIDE CYCLIQUE POUR LE TRAITEMENT DE PHA TYPE 1B

(30) Priority: 14.09.2015 EP 15185093
(43) Date of publication of application: 25.07.2018
(73) Proprietor: APEPTICO Forschung und Entwicklung GmbH, 1150 Wien (AT)
(72) Inventor: WILLAM, Anita, 1100 Wien (AT); LEMMENS-GRUBER, Rosa, 3003 Gablitz (AT); TZOTZOS, Susan, Jane, 1180 Wien (AT); FISCHER, Bernhard, 1160 Wien (AT); SHABBIR, Waheed, 1220 Wien (AT); LUCAS, Rudolf, Martinez, Georgia 30907 (US)
(74) Representative: Schwarz & Partner Patentanwälte OG
(86) International application number: PCT/EP2016/071636
(87) International publication number: WO 2017/046124

(56) References cited:
- WO-A1-2014/173843
- Parmeshwar Shrirang Parmeshwar: "Diplomarbeit: Probing the Binding Sites of PHA1 mutant with the Peptide Drug AP 301", , 1 January 2013 (2013-01-01), pages 1-45, XP055227280, Universität Wien Retrieved from the Internet: URL:http://othes.univie.ac.at/27808/1/2013 -04-29_1148821.pdf [retrieved on 2015-11-10]
- W. SHABBIR ET AL: "Mechanism of Action of Novel Lung Edema Therapeutic AP301 by Activation of the Epithelial Sodium Channel", MOLECULAR PHARMACOLOGY, vol. 84, no. 6, 27 September 2013 (2013-09-27), pages 899-910, XP055227260, DOI: 10.1124/mol.113.089409
- M. LU ET AL: "Small Molecule Activator of the Human Epithelial Sodium Channel", JOURNAL OF BIOLOGICAL CHEMISTRY, vol. 283, no. 18, 11 March 2008 (2008-03-11), pages 11981-11994, XP055082807, ISSN: 0021-9258, DOI: 10.1074/jbc.M708001200
- Anonymous: "Therapeutics | Wellcome Trust", , 6 September 2015 (2015-09-06), XP055227282, Retrieved from the Internet: URL:https://web.archive.org/web/2015090607 2504/http://www.wellcome.ac.uk/Funding/Inn ovations/Funded-projects/Therapeutics/inde x.htm [retrieved on 2015-11-10]

## Description

The present invention relates to cyclic polypeptides and their use in the treatment of autosomal recessive pseudohypoaldosteronism type 1 (PHA type 1B).

### BACKGROUND

Autosomal recessive (AR) pseudohypoaldosteronism type 1, (PHA type1B) is a rare, life-threatening disease that presents in the first few days of life with failure to thrive, weight loss, salt-wasting, hyperkalaemia and metabolic acidosis. The condition was first characterised in 1958 (Cheek & Perry, 1958). Autosomal recessive pseudohypoaldosteronism type 1 is a life-threatening condition in which the sodium ion channel, ENaC, found in kidneys, colon, salivary and sweat glands and in the lung has lost the function to promote sodium ion (Na+ ion) movement across cell layers. Non-function of ENaC results in loss of sodium in the urine and faeces and severe salt imbalance in the body. Characteristic features are low levels of sodium (hyponatremia) and high levels of potassium (hyperkalemia) in the blood. The disorder involves multiple organ systems and is therefore also referred to as systemic PHA type 1 to distinguish it from the milder, autosomal dominant (AD) or renal PHA type 1, in which salt loss is mainly restricted to the kidney and which is caused by mutations in a different gene (Riepe, 2009). Differentiation between renal and systemic PHA type 1 may be made based on Na requirements, ease of management of electrolyte imbalance, sweat test results and genetic testing (Amin et al, 2013). Notably, children with systemic, but not renal pseudohypoaldosteronism have frequent lower respiratory tract illnesses of unknown cause and are often misdiagnosed as suffering from cystic fibrosis (Hanukoglu et al, 1994; Marthinsen et al, 1998; Huber et al, 2010).

Neonates first diagnosed with PHA type1B have characteristically elevated renin and aldosterone values, but are unable to maintain blood pressure. Laboratory evaluation of PHA typelb patients shows increased plasma renin activity with high serum aldosterone concentrations and hyponatremia as well as hyperkalaemia already mentioned. Aggressive salt replacement and control of hyperkalaemia are necessary to ensure survival.

Autosomal recessive pseudohypoaldosteronism type I (PHA type 1B) is caused by homozygous or compound heterozygous mutation in any one of three genes encoding subunits of the epithelial sodium channel (ENaC) (the alpha subunit, the beta subunit, the gamma subunit).

Due to mutations in any one of three genes encoding for the alpha, beta and gamma subunits of the epithelial sodium channel (ENaC), the amino acid sequence of the mutant expressed ENaC protein is incomplete or otherwise different from the mature (non-mutant) ENaC. Mutant ENaC is virtually inactive and does not promote transport of sodium ions across cells and membranes.

Clinical manifestation of systemic PHA type 1 occurs within the neonatal period with diagnosis usually based on elevated Na concentration in the sweat and absent nasal or rectal transepithelial voltage differences (Riepe, 2009). The clinical phenotype is one of severe renal salt-wasting, hyperkalaemia, metabolic acidosis and elevated plasma renin and aldosterone levels. Children suffering from AR PHA type 1 often show pulmonary complications resulting from reduced Na-dependent liquid absorption and increased volume of airway surface liquid (Kerem et al, 1999). Onset of respiratory symptoms is typically within weeks or months of birth, with persistent rhinorrhea, recurrent ear and sinus infections, chest congestion, cough and tachypnea often associated with fever, wheezing and crackles being frequently observed. During these episodes of respiratory illness, which may occur several times per year, the patient's chest X-ray may show peribronchial thickening, atelectasis and/or small fluffy infiltrates (Thomas et al, 2002). Pulmonary complications can occasionally prove fatal (Sharma et al, 2013).

Autosomal recessive pseudohypoaldosteronism type I is a life-long disease and shows little improvement with time (Zennaro et al, 2004). Patients suffering from PHA type 1B are at risk from life-threatening, salt-losing crises, combined with severe hyperkalaemia and dehydration throughout their entire lives (Riepe, 2009).

Currently, treatment of PHA type 1B is limited to fluid and electrolyte management. Current symptomatic treatment of PHA type 1B is a combination of beta2 agonist therapy to reduce the excess lung fluid and aggressive salt replacement and control of hyperkalaemia to restore electrolyte balance.

### BRIEF DESCRIPTION OF THE INVENTION

Currently there exists no drug-based therapy that restores or at least increases the Na⁺ ion transport capacity of mutant loss-of-function ENaC.

Hence, it is an objective of the present invention to provide a drug-based therapy that restores Na⁺ ion transport capacity of mutant loss-of-function ENaC to physiological level.

This objective is solved by a
cyclic polypeptide comprising at least six contiguous amino acids from the amino acid sequence SEQ ID NO:1
Gly-Gln-Arg-Glu-Thr-Pro-Glu-Gly-Ala-Glu-Ala-Lys-Pro-Trp-Tyr
for the use in the treatment of autosomal recessive pseudohypoaldosteronism type 1 (PHA type1B) and for the restoration of the Na⁺ ion transport capacity of mutated loss-of-function ENaC.

Though there currently does not exist a drug-based therapy that converts mutant, virtually inactive ENaC into physiologically active ENaC it has been found that cyclic polypeptides according to the inventions restore Na⁺ ion transport capacity and to compensate for amino acid mutations of mutant loss-of-function ENaC to normal levels. Though cyclic peptides as detailed below have been described to modulate ENaC activity of "normal" (wild-type active) non-mutant ENaC, it is surprising that the cyclic peptides are also capable of compensatjng for amino acid mutations and restoring mutant loss-of-function ENaC back to normal levels.

In a preferred embodiment the cyclic polypeptide comprises at least nine contiguous amino acids from the amino acid sequence SEQ ID NO:1.

It turned out that cyclic polypeptides comprising the amino acid sequence
Thr-Pro-Glu-Gly-Ala-Glu (= SEQ ID NO:5)
of SEQ ID NO:1 show the strongest binding to the ENaC receptor. Thus, in one embodiment it is preferred that the cyclic polypeptides comprises the amino acid sequence SEQ ID NO:5 Thr-Pro-Glu-Gly-Ala-Glu
of SEQ ID NO:1

In one embodiment the cyclic polypeptide is characterized by the amino acid sequence Gly-Gln-Arg-Glu-Thr-Pro-Glu-Gly-Ala-Glu-Ala-Lys-Pro-Trp-Tyr.

There are different ways to form a ring to provide a cyclic polypeptide according to the invention.
a) In one embodiment the cyclic ring is formed by amide bonds in the polypeptide between the single amino acids to form a peptide back bone and a disulfide bridge between two cysteine amino acids to form a ring. Hence, one embodiment includes a disulfide bond between two cysteine amino acids.
   In that embodiment the polypeptide preferably comprises SEQ ID NO:2
   Cys-Gly-Gln-Arg-Glu-Thr-Pro-Glu-Gly-Ala-Glu-Ala-Lys-Pro-Trp-Tyr-Cys, wherein the disulfide bond is formed between Cys 1 and Cys 17 of SEQ ID NO:2. also referred to as AP301.
b) In one embodiment the cyclic ring is formed by amide bonds in the polypeptide between the single amino acids to form a circular polypeptide. In one embodiment such a cyclic ring includes a non-natural amino acid such as γ-aminobutyric acid (GABA). also referred to as AP318.

Most preferably in all embodiments as mentioned above the cyclic polypeptide is characterized in that the cyclic ring comprises at least 10, preferably at least 14 amino acids of SEQ ID NO:1.

One aspect of the present disclosure relates to a pharmaceutical composition comprising a cyclic polypeptide as mentioned above.

The present invention relates to a pharmaceutical composition containing the cyclic peptide according to the present invention (or a mixture of peptides according to the present invention) and pharmaceutical carrier molecules. This pharmaceutical composition is used for the treatment of PHA type 1B.

The term "pharmaceutical composition" refers to any composition or preparation that contains a cyclic peptide, as defined above, which restore Na⁺ ion transport capacity of mutant loss-of-function ENaC to normal levels. In particular, the expression "a pharmaceutical composition" refers to a composition comprising a cyclic peptide according to the present invention and pharmaceutically acceptable carrier molecules or excipient (both terms are used interchangeably). Suitable carriers or excipients are known to the person skilled in the art, for example saline, Ringer's solution, dextrose solution, buffers, Hank solution, vesicle forming compounds, fixed oils, ethyl oleate, dextrose in saline, substances that enhance iso-tonicity and chemical stability, buffers and preservatives. Other suitable carriers include any carrier that does not itself induce the production of antibodies in the patient that are harmful for the patient. Examples are well tolerable proteins, polysaccharides, polylactic acids, polyglycolic acid, polymeric amino acids and amino acid copolymers. This pharmaceutical composition can (as a drug) be administered via appropriate procedures known to the skilled person. The preferred route of administration is pulmonary inhalation as aerosol or intravenous administration. For parenteral administration, the pharmaceutical composition of the present invention is provided in injectable dosage unit form, eg as a solution, suspension or emulsion, formulated in conjunction with the above-defined pharmaceutically acceptable excipients. The dosage and method of administration, however, depends on the individual patient to be treated. In general, the peptide according to the present invention is administered at a dose of between 1 µg/kg and 10 mg/kg, more preferably between 10 µg/kg and 5 mg/kg, most preferably between 0.1 and 2 mg/kg. Preferably, the composition will be administered as an intraperitoneal bolus dosage. Also continuous infusion can be applied. In this case, the peptide is delivered at a dose of 5 to 20 µg/kg/minute, more preferably 7-15 µg/kg/minute infusion. For pulmonary inhalation as aerosol, the pharmaceutical composition of the present invention is provided as dry powder or liquid preparation in suitable dosage unit form, e.g. as dry powder particles prepared by lyophilisation and/or spray drying, as solution, suspension and emulsion, formulated in conjunction with the above-defined pharmaceutically acceptable excipients. Aerosol particles suitable of pulmonary inhalation, either dry powder particles or liquid aerosol particles, have particles diameters below 50 µm, more preferably below 10 µm. Dry powder particles can be inhaled by dry powder inhalers. Liquid particles can be inhaled by nebulisers. The dosage and method of administration for pulmonary inhalation, however, depends on the individual patient to be treated. In general, the peptide according to the present invention is administered at a dose of between 1 µg/kg and 10 mg/kg, more preferably between 10 µg/kg and 5 mg/kg, most preferably between 0.1 and 2 mg/kg. Preferably, the composition will be administered as repeated inhalation dosages. Also continuous inhalation can be applied. In this case, the peptide is delivered at a dose of 5 to 20 µg/kg/minute, more preferably 7-15 µg/kg/minute.

One aspect of the present disclosure relates to a method of treating a patient suffering from autosomal recessive pseudohypoaldosteronism type 1 (PHA type1B) comprising a cyclic peptide as mentioned above.

The amiloride-sensitive epithelial sodium ion channel (ENaC)
Functionally active ENaC is usually composed of one or two alpha or delta subunits together with a beta and a gamma unit. Each ENaC polypeptide chain is composed of short -NH₂ and-COOH termini located intracellularly and two transmembrane regions on either side of a large extracellular loop domain.

The ENaC is located in the apical membrane of polarised epithelial cells of the lung, distal colon, distal nephron, sweat and salivary glands, and other organs and tissues. In polarised tight epithelia ENaC is the rate-limiting step for Na⁺ ion absorption; abnormal ENaC function disturbs salt and water homeostasis and the physiological operation of organs and tissues in which it occurs (Garty & Palmer, 1997; Kellenberger & Schild, 2002). Transepithelial transport of Na⁺ ion through a cell may be described as a two-step process, the driving force for which is provided by the large electrochemical gradient for Na⁺ ion existing across the apical membrane. Functional active ENaC mediates entry of Na⁺ ions from the apical side of the membrane. This apical entry of Na⁺ through ENaC can be blocked by application of submicromolar concentrations of amiloride.

In the mammalian lung, regulation of Na+ ion transport is crucial to maintaining an optimal level of alveolar lining fluid necessary for efficient gas exchange (Eaton et al, 2009).

Mutations either within ENaC genes or in upstream regulatory regions disrupt normal ENaC expression resulting in dysfunction and aberrant regulation of the channel.

PHA type 1B patients carry loss-of-function mutations in the alpha ENaC subunit, with mutations in the beta and gamma subunits being less frequently observed.

The original AP301 peptide mimics the lectin-like or TIP domain of TNF-alpha, corresponding to residues C101-E116 of wild type human TNF (Lucas et al, 1994). In AP301, cyclo(CGQRETPEGAEAKPWYC), theoretical average molecular mass 1923.1, C101 has been replaced by glycine and E116 by cysteine, an N-terminal cysteine is added and the sequence of amino acid residues representing the lectin-like domain is constrained into a cyclic structure via a disulphide bond between the side chains of the terminal cysteine residues. Cyclisation is achieved by oxidation of the terminal cysteine residues to form a disulphide bridge.

AP318 Cyclo(4-aminobutanoic acid-GQRETPEGAEAKPWYD), theoretical average molecular mass 1901.0, is a TIP peptide in which cyclisation is achieved by creating an amide bond between the amino group of N-terminal 4-aminobutanoic acid and the side chain carboxyl group attached to the Beta-carbon of the C-terminal aspartic acid residue.

AP301
[CGQRETPEGAEAKPWYC] (cyclo Cbeta1-Cbeta17)

AP318
[Gaba-GQRETPEGAEAKPWYD-OH] (cyclo 1-Dγ17)

### DETAILED DESCRIPTION

Further details of the invention are depicted in the figures and their description.

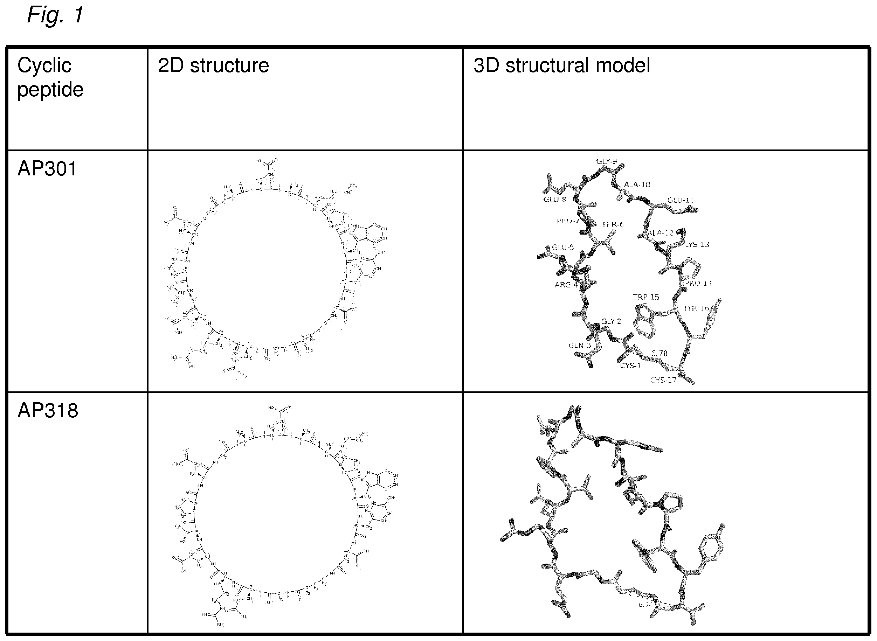

- Fig. 1: shows a comparison of peptides AP301 and AP318.

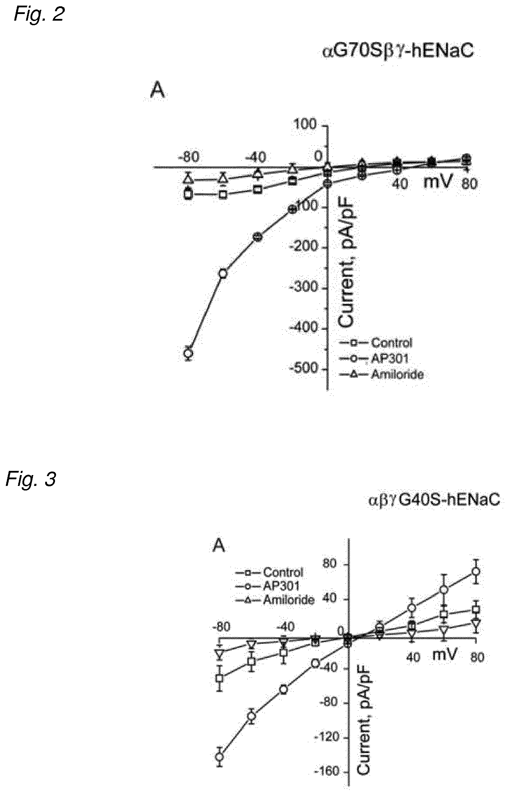

- Fig.2: shows the effect of AP301 on αG70Sβγ-hENaC. Whole cell I/V relationship of HEK-293 cells transiently expressing αG70Sβγ-hENaC plotted for control, in presence of 240 nM AP301 and after addition of 10 µM amiloride.
- Fig. 3: shows the effect of AP301 on αβγG40S-hENaC. Whole cell I/V relationship of HEK-293 cells transiently expressing αβγG40S-hENaC plotted for control, in presence of 240 nM AP301 and after addition of 10 µM amiloride.

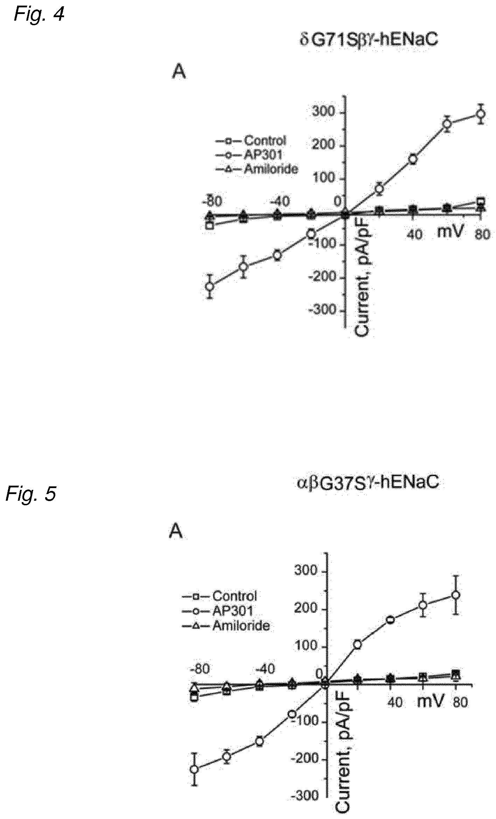

- Fig. 4: shows the effect of AP301 on δG71Sβγ-hENaC. Whole cell I/V relationship of HEK-293 cells transiently expressing δG71Sβγ-hENaC plotted for control, in presence of 240 nM AP301 and after addition of 10 µM amiloride.
- Fig. 5: shows the effect of AP301 on PHA type Ib mutant αβG37Sγ-hENaC. Whole cell I/V relationship of HEK-293 cells transiently expressing αβG37Sγ-hENaC plotted for control, in presence of 240 nM AP301 and after addition of 10 µM amiloride.
- Fig. 6: shows bar graphs of amiloride-sensitive inward sodium currents. HEK-293 cells transiently transfected with indicated mutant subunits were patched in the whole cell mode; inward current was elicited at -100 mV. 200nM of AP318 or AP301 were applied.

### Synthesis of cyclic peptides

All peptides are synthesised by solid-phase methods; they have been designed to retain the native conformation of the lectin-like domain as much as possible whilst at the same time exploring alternative linking solutions to bring about cyclisation of the linear sequence.

Peptides are synthesised by solid-phase peptide synthesis according to the fluorenylmethyloxycarbonyl/t-butyl protection strategy on 2-chlorotritylchloride resin. Diisopropyl carbodiimide and N-hydroxybenzotriazole are used as coupling reagents. All coupling steps are carried out in N-N-dimethyl formamide. Protected amino acids are coupled in succession to the peptide chain, starting with the C-terminal amino acid. Deprotection of fluorenylmethoxycarbonyl is carried out in 20% piperidine in N-N-dimethyl formamide. Cleavage of the completed, partially-protected peptide from the resin is carried out in a 1:1 mixture of acetic acid and dichloromethane. In the case of solnatide and mutant TIP peptide, after cleavage from the resin, side-chain deprotection in 95% trifluoroacetic acid, 5% water, is carried out followed by cyclisation by oxidation of terminal cysteine residues, achieved by aeration of the crude linear peptide at pH 8.5 for 90 hours. Crude peptide product is purified by reverse phase medium pressure liquid chromatography (RP-MPLC) on an RP-C18-silica gel column with a gradient of 5% - 40% acetonitrile. Finally, the trifluoracetate counter-ion is replaced by acetate on a Lewatit MP64 column (acetate form). Following a final wash in water, the purified peptide as acetate salt is lyophilised and obtained as a white to off-white powder. In the case of cysteine-free peptides, the cyclisation step is carried out on the partially-protected linear peptide following cleavage from the 2-chlorotritylchloride resin. After selective cyclisation of the cysteine-free peptides, side-chain deprotection in trifluoroacetic acid followed by preparative RP-MPLC, replacement of the trifluoroacetate ion by acetate and lyophilisation of the acetate form of the peptide was carried out as for cysteine-containing peptides. In the case of AP318, in which cyclisation involves amide bond formation through the side chain carboxyl group of aspartic acid, selective cyclisation is achieved by starting the synthesis using the C-terminal aspartic acid N-protected with the fluorenylmethyloxycarbonyl group and with the C-alpha carboxyl protected with a tertiary butyl (OtBu) group. Synthesis proceeds by linkage of the C-terminal aspartic acid residue to the trityl resin through the side chain carboxyl group, followed by stepwise addition of the protected amino acid residues to the peptide chain. After deprotection of the amino group of N-terminal 4-aminobutanoic acid and cleavage of the side-chain protected peptide from the resin, cyclisation is carried out through the free side-chain carboxyl group and the amino group of N-terminal 4-aminobutanoic acid. Finally, side chain protecting groups are removed with trifluoracetic acid and the peptide purified by RP-MPLC as for the other peptides.

Molecular masses of the peptides are confirmed by electrospray ionisation mass spectrometry or MALDI-TOF-MS and their purity determined by analytical high performance liquid chromatography.

### Electrophysiological assay of AP301 and AP318 activation of endogenously and heterologously-expressed ENaC

The ENaC-activating properties of cyclic peptides are tested electrophysiologically in vitro using whole cell and single cell patch clamp techniques Hazemi et al, 2010; Tzotzos et al, 2013; Shabbir et al, 2013). A whole call patch clamp assay is used to measure the induced amiloride-sensitive Na+ current, calculate concentration-response curves and thus estimate the potency measured as effective concentration at half maximum response (EC50), for cyclic peptides.
Patch clamp experiments are performed to estimate the potency of AP301 / AP318 on transiently expressed αβγ-hENaC in HEK-293, CHO cells and A549 cells

### Summary of the rationale for investigating the potential application of AP301 and AP318 for treatment of pulmonary symptoms of patients suffering from PHA type 1b

Pseudohypoaldosteronism type 1B (PHA type 1B) is caused by loss-of-function mutations in the genes encoding the amiloride-sensitive epithelial sodium channel (ENaC). The condition presents in newborns as life-threatening severe dehydration, hyponatremia and hyperkalaemia due to sodium loss involving kidneys, colon, lungs and sweat and salivary glands; children suffer from pulmonary ailments because reduced sodium-dependent liquid absorption results in elevated lung liquid levels. The disease shows no improvement with age and patients require life-long salt supplements and dietary manipulation to reduce potassium levels.

AP301 and AP318 can be applied in a patch clamp assay to test the response of cells heterologously-expressing human ENaC subunits into which loss-of-function mutations known to cause PHA type 1B have been introduced by site-directed mutagenesis. In this way the ability of AP301 and AP318 to restore the amiloride-sensitive sodium current in cells expressing these mutated loss-of-function ENaC subunits, can be measured. Increase in the sodium current in the presence of cyclic peptides indicates their ability to restore Na⁺ ion movement by loss-of-function ENaC carrying PHA type 1B mutations, to restore ENaC function therefore and their potential as therapies for PHA type 1B patients.

Surprisingly it has been detected that cyclic peptides such as AP301 and AP318 can restore Na⁺ ion transport activity to loss-of-function mutant ENaC. Thus AP301 and AP318 are potential therapies for pulmonary symptoms of PHA type IB.

### Experimental protocol

### In vitro study of effect of AP301 and AP318 on heterologously expressed ENaC carrying PHA type IB mutations

The effect of cyclic peptides on the amiloride-sensitive Na+ current was observed in HEK cells heterologously expressing human ENaC subunits (HEK cells show no endogenous expression of ENaC [Ruffieux-Daidie et al, 2008]) into which single point mutations of alpha, beta and gamma ENaC, the same as those found responsible for the pathological phenotype of patients suffering from PHA type IB, had been introduced by site-directed mutagenesis. In addition, mutant delta ENaC subunits were also constructed by site-directed mutagenesis, containing homologous mutations to those observed in conserved positions in the other three ENaC subunits.

### Construction of ENaC PHA type I mutants and expression in HEK 293

Various types of mutations of ENaC can be reproduced by site directed mutagenesis of wild type ENaC subunit DNA cloned into plasmid vectors.

### Site-directed mutagenesis

Point mutations were introduced into cDNA encoding alpha, beta, gamma and delta ENaC using a commercially available site-directed mutagenesis kit (QuikChange Lightning Site-Directed Mutagenesis Kit; Agilent Technologies). The cDNAs encoding alpha, beta, and gamma-hENaC had been donated by Dr. Peter Snyder (University of Iowa, Carver College of Medicine, Iowa City, IA); cDNA encoding delta-hENaC had been donated by Dr. Mike Althaus (Justus-Liebig University, Giessen, Germany).

Mutagenic primers were designed individually based on descriptions of the individual mutations in the original scientific reports. The primer design program provided on the manufacturer's website was used as a guide and primers themselves were ordered from Sigma-Aldrich. Mutant strands were synthesised by PCR with a Pfu-based DNA polymerase using 100 ng wild-type (WT) cDNA encoding alpha, beta, gamma or delta-hENaC. Parental (WT) strands were removed and the resulting plasmid DNA containing mutated ENaC was transformed into E.coli competent cells. Following growth in culture, plasmid DNA was extracted from the E.coli cells using a commercially available plasmid isolation kit (GeneJET Plasmid Miniprep Kit; Thermoscientific) and isolated by column chromatography. All the mutant constructs were checked by restriction site mapping and sequencing.

### Transfection of HEK-293 cells for heterologous expression of hENaC

HEK-293 cells were transfected with the mutant alpha-, beta-, gamma- and delta-hENaC and WT alpha-, beta-, gamma- and delta-hENaC plasmid DNA using a commercially available kit (X-treme Gene HP transfection reagent (Roche Diagnostics, Mannheim, Germany) following the protocol recommended by the manufacturer. One mutant subunit together with the remaining two WT subunits were transfected simultaneously to give expression of trimeric mutant ENaC. Expression of WT ENaC was achieved by simultaneous transfection with WT alpha-, beta- and gamma-hENaC plasmid DNA or with WT delta-, beta- and gamma-hENaC plasmid DNA.

### Patch clamp testing of ENaC-activating ability of AP301 and AP318 in HEK cells transiently expressing mutant ENaC

Each HEK-293 cell line transiently expressing WT αβγ-hENaC, WT δβγ-hENaC or a mutant hENaC subunit co-expressed with WT subunits, was tested in a whole cell patch clamp assay with AP301 and selected mutants at conserved positions with AP318. Whole cell currents were recorded as previously described (Shabbir et al, 2013).

### Concentration response measurements

Concentration-response curves were plotted, and EC50 values and Hill coefficients were determined using Microcal Origin 7.0. The whole-cell sodium current of HEK-293 cells transiently transfected with WT αβγ-hENaC, WT δβγ-hENaC or a mutant hENaC, was recorded at a holding potential (Eh) of -80 mV following cumulative addition of AP301 stock solution to the bath solution, resulting in final concentrations ranging from 3.5 to 240 nM solnatide. Finally, amiloride was added to enable estimation of the peptide-induced increase in amiloride-sensitive Na+ current. The activity of AP301 was expressed as a percentage of the paired amiloride response, owing to variability in hENaC expression between different batches of cultured cells. Amiloride was used at 10 µM for WT αβγ-hENaC, WT δβγ-hENaC or mutant hENaC; these concentrations yielded greater than 95% hENaC inhibition. Only cells with clear amiloride response were included in data analysis.

### Current-voltage relationships

Whole-cell current-voltage (I/V) relationships of HEK-293 cells transiently infected with αβγ-hENaC, WT δβγ-hENaC or a mutant hENaC were determined for control (before addition of AP301), treatment with 240 nM AP301 and following addition of 10 µM amiloride, respectively. After GOhm-seal (G'Ω-seal) formation, and an equilibration period of 5 min, sodium current was recorded at Eh from -80 to +80 mV in 20 mV increments held for 1 min at each Eh.

### Statistical analysis

Data represent the mean ± S.E. unless otherwise stated; experiments were performed on three to seven batches of independently transfected cells in the HEK-293 heterologous expression system. Statistical significance between different groups was determined using an unpaired, two-tailed Student's t test using GraphPad Prism version 3.02 (GraphPad Software, San Diego).

### RESULTS

**Table 1. Results of AP301 in a whole cell patch clamp assay with HEK-293 cells expressing PHA type IB mutant hENaC and homologues**

| hENaC | Amiloride-sensitive current | AP301 restored Na+ current | EC₅₀ |
|---|---|---|---|
| | (pA) | (pA) | (nM) |
| αβγ WT | 81.2 ± 5.5 | | 54.7 ± 2.2^{c} |
| δβγ WT | 93.5 ± 9.5 | | 46.2 ± 1.5 |
| αG70Sβγ | loss-of- function | 392.1 ± 14.2 | 61.9 ± 2.1 |
| αβG37Sγ^{a} | loss-of-function | 192.8 ± 12.3 | 65.8 ± 3.2 |
| αβγG40S | loss-of-function | 90.8 ± 8.9 | 69.1 ± 2.7 |
| δG71Sβγ | loss-of-function | 185.1 ± 21.2 | 42.9 ± 0.5 |
| αQ101Kβγ^{a} | loss-of- function | 79.1 ± 11.2 | |
| αβQ66Kγ | loss-of- function | 448.5 ± 57.5 | 56.9 ± 16.7 |
| αβγQ70K | loss-of- function | 305.2 ± 10.1 | 61.5 ± 4.0 |
| δQ102Kβγ | loss-of- function | 121.9 ± 14.6 | |
| αC133Yβγ^{a} | loss-of- function | 111.8 ± 10.6 | |
| αβC98Yγ | loss-of- function | 296.3 ± 4.2 | 79.4 ± 1.7 |
| αβγC100Y | loss-of- function | 307.9 ± 15.7 | 88.2 ± 11.9 |
| δC134Yβγ | loss-of- function | 219.1 ± 19.8 | |
| αG327Cβγ^{a} | loss-of-function | 196.8 ± 16.7 | |
| αβG294Cγ | loss-of-function | 76.9 ± 13.5 | |
| αβγG305C | loss-of-function | 125.5 ± 15.7 | |
| δG303Cβγ | loss-of-function | 48.5 ± 8.2 | |

| | | | |
|---|---|---|---|
| (^{a} - mutants observed in patients) | | | |

### Effect of AP301 on PHA type IB αβG37Sγ-hENaC and homologues

The effect of AP301 on HEK-293 cells expressing the PHA type 1b mutant αβG37Sγ-hENaC or one of its lab-constructed homologues is shown in Figures below. Whole-cell current-voltage (I/V) relationships are shown for each mutant loss-of-function hENaC transiently expressed in HEK-293 cells as well as absolute mean values of inward current density at a holding potential of -80 mV during control phase, following addition of 240 nM AP301 and after final addition of amiloride (10 µM) to the bath solution (Figures below).

### Effect of AP318 on PHA type IB mutant ENaC

In order to test whether the activity restoring effect on PHA type 1B mutant ENaC is exclusive to AP301 or whether it is a property of cyclic peptides in general, three PHA type IB mutant ENaCs containing mutations in the α-, β- and γ-hENaC subunits, respectively, were tested in a whole cell patch clamp assay in the presence of AP318 as well AP301. All three mutants have been observed in patients suffering from PHA type 1B and occur at conserved positions in the ENaC subunits; two of these mutants, αQ101Kβγ-hENaC and αβG37Sγ-hENaC had been previously tested with AP301. The third mutant occurs in the γ subunit: αβγV543fs-hENaC and in contrast to all mutants so far tested is a frameshift mutant resulting in a truncated γ subunit.

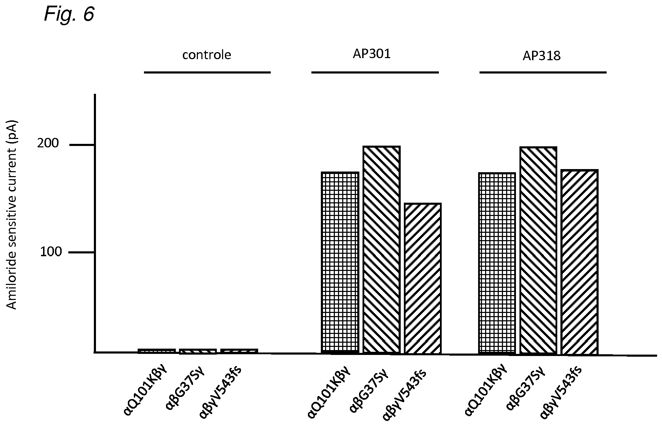

The results of testing HEK-293 cells expressing these mutant ENaCs in a whole cell patch clamp assay in the presence of AP318 and AP301 are shown in Table 2 and Figure 6.

**Table 2. Amiloride-sensitive current in a whole cell patch clamp assay of HEK-293 cells transiently expressing WT ENaC and ENaC containing PHA type 1b mutations in the absence (Control) and presence of AP301 and AP318 (mean ± SE values for inward current measured in pA, n = 5; peptide concentration 220 nM).**

| ENaC | No peptide | AP301 | AP318 |
|---|---|---|---|
| | Inward current (pA) | Inward current measured (pA) | |
| | | Peptide concentration 220 nM | |
| WT αβγ-hENaC | 67.2±5.2 | | |
| αQ101Kβγ-hENaC | loss-of-function | 174.7±8.7 | 176.7±6.9 |
| αβG37Sγ-hENaC | loss-of-function | 189.8±7.1 | 191.8±5.8 |
| αβγV543fs-hENaC | loss-of-function | 143.3±8.2 | 177.7±6.5 |

### The following conclusions can be drawn from the results obtained so far:

1) PHA type IB mutations resulted in a loss-of-function in the amiloride-sensitive sodium current through ENaC compared to WT hENaC.
2) AP301 restored Na⁺ ion transport capacity and compensated for amino acid mutations of all the mutant loss-of-function hENaCs observed in PHA type IB patients: αβG37Sγ-, αQ101Kβγ- and αG327Cβγ-hENaC.
3) Compared to the physiological level of amiloride-sensitive sodium ion current observed with WT αβγ- and δβγ-hENaC, AP301 restored amiloride-sensitive sodium ion current of mutant loss-of-function ENaC to levels comparable to non-mutant active ENaC.
4) Concentration-response curves and EC50 values for PHA type IB mutant αβG37Sγ-hENaC and corresponding homologues indicate that AP301 has the potential to restore activity and to compensate amino acid mutations in all of these mutant ENaC channels, which have loss-of-function compared to WT αβγ- and δβγ-hENaC
5) Current-voltage (I/V) relationships for PHA type IB mutant αβG37Sγ-hENaC and corresponding homologues characterise the restoring effect of AP301 on these mutant channels in greater detail. The same mutation occurring at a conserved position in the different subunits of hENaC, results in an sodium ion channel with different functional properties and phenotypic effect, as clearly reflected by the restored activity in the presence of AP301 (Table 3).
6) Results of the whole cell electrophysiological assay in the absence of AP301 and AP318 show that, compared to wild type, PHA type 1B mutations in all αβγ-hENaC subunits result in loss-of-function. In the presence of AP301 and AP318, significantly increased amiloride-sensitive sodium ion current through PHA-1B mutants demonstrating restoration of normal sodium ion channel function was observed.

### Overall Conclusion

It can be concluded from these results that AP301 and AP318 can restore Na⁺ ion transport and compensate for amino acid mutations in loss-of-function PHA type IB mutant hENaCs, indicating the potential of cyclic peptides to restore impaired ENaC function and to compensate for amino acid mutations in these mutants and thereby act as a therapy to treat patients suffering from systemic PHA type I.

### References

1. Aberer E, Gebhart W, Mainitz M, Pollak A, Reichel G, Scheibenreiter S. [Sweat glands in pseudohypoaldosteronism]. Hautarzt. 1987 Aug;38(8):484-7. German. PubMed PMID: 3654220.
2. Adachi M, Tachibana K, Asakura Y, Abe S, Nakae J, Tajima T, Fujieda K. Compound heterozygous mutations in the gamma subunit gene of ENaC (1627delG and 1570-1G->A) in one sporadic Japanese patient with a systemic form of pseudohypoaldosteronism type 1. J Clin Endocrinol Metab. 2001 Jan;86(1):9-12. PubMed PMID: 11231969.
3. Akkurt I, Kuhnle U, Ringenberg C. Pseudohypo-aldosteronism and cholelithiasis: coincidence or pathogenetic correlation? Eur J Pediatr. 1997 May;156(5):363-6. PubMed PMID: 9177977.
4. Althaus M, Clauss WG, Fronius M. Amiloride-sensitive sodium channels and pulmonary edema. Pulm Med. 2011;2011:830320. Epub 2010 Dec 29. PubMed PMID: 21637371.
5. Amin N, Alvi NS, Barth JH, Field HP, Finlay E, Tyerman K, Frazer S, Savill G, Wright NP, Makaya T, Mushtaq T. Pseudohypoaldosteronism type 1: clinical features and management in infancy. Endocrinol Diabetes Metab Case Rep. 2013;2013:130010. doi: 10.1530/EDM-13-0010. Epub 2013 Aug 30. PubMed PMID: 24616761; PubMed Central PMCID: PMC3922296.
6. Baconguis I, Bohlen CJ, Goehring A, Julius D, Gouaux E. X-ray structure of acid-sensing ion channel 1 snake toxin complex reveals open state of a Na(+)-selective channel. Cell. 2014 Feb 13;156(4):717-29. doi: 10.1016/j.cell.2014.01.011. Epub 2014 Feb 6. PubMed PMID: 24507937; PubMed Central PMCID: PMC4190031.
7. Belot A, Ranchin B, Fichtner C, Pujo L, Rossier BC, Liutkus A, Morlat C, Nicolino M, Zennaro MC, Cochat P. Pseudohypoaldosteronisms, report on a 10-patient series. Nephrol Dial Transplant. 2008 May;23(5):1636-41. doi: 10.1093/ndt/gfm862. PubMed PMID: 18424465.
8. Bonny O, Chraibi A, Loffing J, Jaeger NF, Gründer S, Horisberger JD, Rossier BC. Functional expression of a pseudohypoaldosteronism type I mutated epithelial Na+ channel lacking the pore-forming region of its alpha subunit. J Clin Invest. 1999 Oct;104(7):967-74. PubMed PMID: 10510337; PubMed Central PMCID: PMC408554.
9. Bonny O, Knoers N, Monnens L, Rossier BC. A novel mutation of the epithelial Na+ channel causes type 1 pseudohypoaldosteronism. Pediatr Nephrol. 2002 Oct;17(10):804-8. Epub 2002 Aug 21. PubMed PMID: 12376807.
10. Bonny O, Rossier BC. Disturbances of Na/K balance: pseudohypoaldosteronism revisited. J Am Soc Nephrol. 2002 Sep;13(9):2399-414. Review. PubMed PMID: 12191985.
11. Braun C, Hamacher J, Morel DR, Wendel A, Lucas R. Dichotomal role of TNF in experimental pulmonary edema reabsorption. J Immunol. 2005 Sep 1;175(5):3402-8. PubMed PMID: 16116234.
12. Canessa CM, Schild L, Buell G, Thorens B, Gautschi I, Horisberger JD, Rossier BC. (B) Amiloride-sensitive epithelial Na+ channel is made of three homologous subunits. Nature. 1994 Feb 3;367(6462):463-7. PubMed PMID: 8107805.
13. Chang SS, Grunder S, Hanukoglu A, Rösler A, Mathew PM, Hanukoglu I, Schild L, Lu Y, Shimkets RA, Nelson-Williams C, Rossier BC, Lifton RP. Mutations in subunits of the epithelial sodium channel cause salt wasting with hyperkalaemic acidosis, pseudohypoaldosteronism type 1. Nat Genet. 1996 Mar;12(3):248-53. PubMed PMID: 8589714.
14. Cheek DB, Perry JW. A salt wasting syndrome in infancy. Arch Dis Child. 1958 Jun;33(169):252-6. PubMed PMID: 13545877; PubMed Central PMCID: PMC2012226.
15. Czikora I, Alli A, Bao HF, Kaftan D, Sridhar S, Apell HJ, Gorshkov B, White R, A, Wendel A, Pauly-Evers M, Hamacher J, Garcia-Gabay I, Fischer B, Verin A, Bagi Z, Pittet JF, Shabbir W, Lemmens-Gruber R, Chakraborty T, Lazrak A, Matthay MA, Eaton DC, Lucas R. A Novel TNF-mediated Mechanism of Direct Epithelial Sodium Channel Activation. Am J Respir Crit Care Med. 2014 Jul 16. PubMed PMID: 25029038.
16. Dirlewanger M, Huser D, Zennaro MC, Girardin E, Schild L, Schwitzgebel VM. A homozygous missense mutation in SCNN1A is responsible for a transient neonatal form of pseudohypoaldosteronism type 1. Am J Physiol Endocrinol Metab. 2011 Sep;301(3):E467-73. doi: 10.1152/ajpendo.00066.2011. Epub 2011 Jun 7. PubMed PMID: 21653223.
17. Dogan CS, Erdem D, Mesut P, Merve A, Sema A, Iffet B, Afig B. A novel splice site mutation of the beta subunit gene of epithelial sodium channel (ENaC) in one Turkish patient with a systemic form of pseudohypoaldosteronism Type 1. J Pediatr Endocrinol Metab. 2012;25(9-10):1035-9. doi: 10.1515/jpem-2012-0083. PubMed PMID: 23426840.
18. Dulebo A, Ettrich R, Lucas R, Kaftan D. A computational study of the oligosaccharide binding sites in the lectin-like domain of Tumor Necrosis Factor and the TNF-derived TIP peptide. Curr Pharm Des. 2012;18(27):4236-43. PubMed PMID: 22697478; PubMed Central PMCID: PMC3495565.
19. Eaton DC, Helms MN, Koval M, Bao HF, Jain L. The contribution of epithelial sodium channels to alveolar function in health and disease. Annu Rev Physiol.2009;71:403-23. doi: 10.1146/annurev.physiol.010908.163250. Review. PubMed PMID: 18831683.
20. Edelheit O, Hanukoglu I, Gizewska M, Kandemir N, Tenenbaum-Rakover Y, Yurdakök M, Zajaczek S, Hanukoglu A. Novel mutations in epithelial sodium channel (ENaC) subunit genes and phenotypic expression of multisystem pseudohypoaldosteronism. Clin Endocrinol (Oxf). 2005 May;62(5):547-53. Review. PubMed PMID: 15853823.
21. Edelheit O, Hanukoglu I, Shriki Y, Tfilin M, Dascal N, Gillis D, Hanukoglu A.Truncated beta epithelial sodium channel (ENaC) subunits responsible for multi-system pseudohypoaldosteronism support partial activity of ENaC. J Steroid Biochem Mol Biol. 2010 Mar;119(1-2):84-8. doi: 10.1016/j.jsbmb.2010.01.002. Epub 2010 Jan 12. PubMed PMID: 20064610.
22. Ekinci Z, Aytac MB, Cheong HI. A case of SCNN1A splicing mutation presenting as mild systemic pseudohypoaldosteronism type 1. J Pediatr Endocrinol Metab. 2013;26(11-12):1197-200. doi: 10.1515/jpem-2013-0053. PubMed PMID: 23813355.
23. Elia N, Tapponnier M, Matthay MA, Hamacher J, Pache JC, Brundler MA, Totsch M, De Baetselier P, Fransen L, Fukuda N, Morel DR, Lucas R. Functional identification of the alveolar edema reabsorption activity of murine tumor necrosis factor-alpha. Am J Respir Crit Care Med. 2003 Nov 1;168(9):1043-50. Epub 2003 Jul 3. PubMed PMID: 12842853.
24. Fukuda N, Jayr C, Lazrak A, Wang Y, Lucas R, Matalon S, Matthay MA. Mechanisms of TNF-alpha stimulation of amiloride-sensitive sodium transport across alveolar epithelium. Am J Physiol Lung Cell Mol Physiol. 2001 Jun;280(6):L1258-65. PubMed PMID: 11350806.
25. Garty H, Palmer LG. Epithelial sodium channels: function, structure, and regulation. Physiol Rev. 1997 Apr;77(2):359-96. Review. PubMed PMID: 9114818.
26. Geller DS, Rodriguez-Soriano J, Vallo Boado A, Schifter S, Bayer M, Chang SS, Lifton RP. Mutations in the mineralocorticoid receptor gene cause autosomal dominant pseudohypoaldosteronism type I. Nat Genet. 1998 Jul;19(3):279-81. PubMed PMID: 9662404.
27. Gonzales EB, Kawate T, Gouaux E. Pore architecture and ion sites in acid-sensing ion channels and P2X receptors. Nature. 2009 Jul 30;460(7255):599-604. doi: 10.1038/nature08218. PubMed PMID: 19641589; PubMed Central PMCID: PMC2845979.
28. Gründer S, Firsov D, Chang SS, Jaeger NF, Gautschi I, Schild L, Lifton RP, Rossier BC. A mutation causing pseudohypoaldosteronism type 1 identifies a conserved glycine that is involved in the gating of the epithelial sodium channel. EMBO J. 1997 Mar 3;16(5):899-907. PubMed PMID: 9118951; PubMed Central PMCID: PMC1169690.
29. Güran T, Deǧirmenci S, Bulut iK, Say A, Riepe FG, Güran Ö. Critical points in the management of pseudohypoaldosteronism type 1. J Clin Res Pediatr Endocrinol. 2011;3(2):98-100. doi: 10.4274/jcrpe.v3i2.20. Epub 2011 Jun 8. PubMed PMID: 21750640; PubMed Central PMCID: PMC3119449.
30. Hamacher J, Stammberger U, Roux J, Kumar S, Yang G, Xiong C, Schmid RA, Fakin RM, Chakraborty T, Hossain HM, Pittet JF, Wendel A, Black SM, Lucas R. The lectin-like domain of tumor necrosis factor improves lung function after rat lung transplantation--potential role for a reduction in reactive oxygen species generation. Crit Care Med. 2010 Mar;38(3):871-8. PubMed PMID: 20081530.
31. Hanukoglu A, Bistritzer T, Rakover Y, Mandelberg A. Pseudohypoaldosteronism with increased sweat and saliva electrolyte values and frequent lower respiratory tract infections mimicking cystic fibrosis. J Pediatr. 1994 Nov;125(5 Pt 1):752-5. PubMed PMID: 7965429.
32. Hanukoglu A, Edelheit O, Shriki Y, Gizewska M, Dascal N, Hanukoglu I. Renin-aldosterone response, urinary Na/K ratio and growth in pseudohypoaldosteronism patients with mutations in epithelial sodium channel (ENaC) subunit genes. J Steroid Biochem Mol Biol. 2008 Sep;111(3-5):268-74. doi: 10.1016/j.jsbmb.2008.06.013. Epub 2008 Jun 26. PubMed PMID: 18634878.
33. Hartmann EK, Boehme S, Duenges B, Bentley A, Klein KU, Kwiecien R, Shi C, Szczyrba M, David M, Markstaller K. An inhaled tumor necrosis factor-alpha-derived TIP peptide improves the pulmonary function in experimental lung injury. Acta Anaesthesiol Scand. 2013 Mar;57(3):334-41. doi: 10.1111/aas.12034. Epub 2012 Dec 6. PubMed PMID: 23216436.
34. Hartmann EK, Thomas R, Liu T, Stefaniak J, Ziebart A, Duenges B, Eckle D,Markstaller K, David M. TIP peptide inhalation in experimental acute lung injury: effect of repetitive dosage and different synthetic variants. BMC Anesthesiol. 2014 May 26;14:42. doi: 10.1186/1471-2253-14-42. eCollection 2014. PubMed PMID: 24904234; PubMed Central PMCID: PMC4046002.
35. Hazemi P, Tzotzos SJ, Fischer B, Andavan GS, Fischer H, Pietschmann H, Lucas R, Lemmens-Gruber R. Essential structural features of TNF-α lectin-like domain derived peptides for activation of amiloride-sensitive sodium current in A549 cells. J Med Chem. 2010 Nov 25;53(22):8021-9. doi: 10.1021/jm100767p. Epub 2010 Oct 27. PubMed PMID: 20979368; PubMed Central PMCID: PMC2996585.
36. Hribar M, Bloc A, van der Goot FG, Fransen L, De Baetselier P, Grau GE, Bluethmann H, Matthay MA, Dunant Y, Pugin J, Lucas R. The lectin-like domain of tumor necrosis factor-alpha increases membrane conductance in microvascular endothelial cells and peritoneal macrophages. Eur J Immunol. 1999 0ct;29(10):3105-11. PubMed PMID: 10540321.
37. Huber R, Krueger B, Diakov A, Korbmacher J, Haerteis S, Einsiedel J, Gmeiner P, Azad AK, Cuppens H, Cassiman JJ, Korbmacher C, Rauh R. Functional characterization of a partial loss of-function mutation of the epithelial sodium channel (ENaC) associated with atypical cystic fibrosis. Cell Physiol Biochem. 2010;25(1): 145-58. doi: 10.1159/000272059. Epub 2009 Dec 22. PubMed PMID: 20054153.
38. Hummler E, Barker P, Gatzy J, Beermann F, Verdumo C, Schmidt A, Boucher R, Rossier BC. Early death due to defective neonatal lung liquid clearance in alpha-ENaC-deficient mice. Nat Genet. 1996 Mar;12(3):325-8. PubMed PMID: 8589728.
39. Huppmann S, Lankes E, Schnabel D, Bührer C. Unimpaired postnatal respiratory adaptation in a preterm human infant with a homozygous ENaC-α unit loss-of-function mutation. J Perinatol. 2011 Dec;31(12):802-3. doi: 10.1038/jp.2011.46. PubMed PMID: 22124517.
40. Jain L, Chen XJ, Ramosevac S, Brown LA, Eaton DC. (2001) Expression of highly selective sodium channels in alveolar type II cells is determined by culture conditions. Am J Physiol Lung Cell Mol Physiol. 280:L646-58.
41. Jasti J, Furukawa H, Gonzales EB, Gouaux E. Structure of acid-sensing ion channel 1 at 1.9 A resolution and low pH. Nature. 2007 Sep 20;449(7160):316-23. PubMed PMID: 17882215.
42. Kala Ahluwalia G, Dasouki M, Lennon A. Phenotypic variation of autosomal recessive pseudohypoaldosteronism type I: a case in point. Clin Case Rep. 2014 Dec;2(6):326-30. doi: 10.1002/ccr3.129. Epub 2014 Sep 15. PubMed PMID: 25548639; PubMed Central PMCID: PMC4270719.
43. Kashlan OB, Adelman JL, Okumura S, Blobner BM, Zuzek Z, Hughey RP, Kleyman TR, Grabe M. Constraint-based, homology model of the extracellular domain of the epithelial Na+ channel α subunit reveals a mechanism of channel activation by proteases. J Biol Chem. 2011 Jan 7;286(1):649-60. doi: 10.1074/jbc.M110.167098. Epub 2010 Oct 25. PubMed PMID: 20974852; PubMed Central PMCID: PMC3013024.
44. Kellenberger S, Schild L. Epithelial sodium channel/degenerin family of ion channels: a variety of functions for a shared structure. Physiol Rev. 2002 Jul;82(3):735-67. Review. PubMed PMID: 12087134.
45. Kerem E, Bistritzer T, Hanukoglu A, Hofmann T, Zhou Z, Bennett W, MacLaughlin E, Barker P, Nash M, Quittell L, Boucher R, Knowles MR. Pulmonary epithelial sodium-channel dysfunction and excess airway liquid in pseudohypoaldosteronism. N Engl J Med. 1999 Jul 15;341(3):156-62. PubMed PMID: 10403853.
46. Kosari F, Sheng S, Li J, Mak DO, Foskett JK, Kleyman TR. Subunit stoichiometry of the epithelial sodium channel. J Biol Chem. 1998 May 29;273(22):13469-74. PubMed PMID: 9593680.
47. Krueger B, Schlotzer-Schrehardt U, Haerteis S, Zenkel M, Chankiewitz VE, Amann KU, Kruse FE, Korbmacher C. Four subunits (αβγδ) of the epithelial sodium channel (ENaC) are expressed in the human eye in various locations. Invest Ophthalmol Vis Sci. 2012 Feb 2;53(2):596-604. doi: 10.1167/iovs. 11-8581. PubMed PMID: 22167092.
48. Lazrak A, Samanta A, Venetsanou K, Barbry P, Matalon S. Modification of biophysical properties of lung epithelial Na(+) channels by dexamethasone. Am J Physiol Cell Physiol. 2000 Sep;279(3):C762-70. PubMed PMID: 10942727.
49. Lifton RP, Gharavi AG, Geller DS. Molecular mechanisms of human hypertension. Cell. 2001 Feb 23;104(4):545-56. Review. PubMed PMID: 11239411.
50. Lingueglia E, Voilley N, Waldmann R, Lazdunski M, Barbry P. Expression cloning of an epithelial amiloride-sensitive Na+ channel. A new channel type with homologies to Caenorhabditis elegans degenerins. FEBS Lett. 1993 Feb 22;318(1):95-9. PubMed PMID: 8382172.
51. Lucas R, Magez S, De Leys R, Fransen L, Scheerlinck JP, Rampelberg M, Sablon E, De Baetselier P. Mapping the lectin-like activity of tumor necrosis factor. Science. 1994 Feb 11;263(5148):814-7. PubMed PMID: 8303299.
52. Lucas R, Yang G, Gorshkov BA, Zemskov EA, Sridhar S, Umapathy NS, Jezierska-Drutel A, Alieva IB, Leustik M, Hossain H, Fischer B, Catravas JD, Verin AD, Pittet JF, Caldwell RB, Mitchell TJ, Cederbaum SD, Fulton DJ, Matthay MA, Caldwell RW, Romero MJ, Chakraborty T. Protein kinase C-α and arginase I mediate pneumolysin-induced pulmonary endothelial hyperpermeability. Am J Respir Cell Mol Biol. 2012 Oct;47(4):445-53. doi: 10.1165/rcmb.2011-0332OC. Epub 2012 May 10. PubMed PMID: 22582175; PubMed Central PMCID: PMC3488628.
53. Marquardt A, Bernevic B, Przybylski M. Identification, affinity characterisation and biological interactions of lectin-like peptide-carbohydrate complexes derived from human TNF-alpha using high-resolution mass spectrometry. J Pept Sci. 2007 Dec;13(12):803-10. PubMed PMID: 17918767.
54. Marthinsen L, Kornfält R, Aili M, Andersson D, Westgren U, Schaedel C. Recurrent Pseudomonas bronchopneumonia and other symptoms as in cystic fibrosis in a child with type I pseudohypoaldosteronism. Acta Paediatr. 1998 Apr;87(4):472-4. PubMed PMID: 9628311.
55. McDonald FJ, Yang B, Hrstka RF, Drummond HA, Tarr DE, McCray PB Jr, Stokes JB, Welsh MJ, Williamson RA. Disruption of the beta subunit of the epithelial Na+ channel in mice: hyperkalaemia and neonatal death associated with a pseudohypoaldosteronism phenotype. Proc Natl Acad Sci U S A. 1999 Feb 16;96(4):1727-31. PubMed PMID: 9990092; PubMed Central PMCID: PMC15575.
56. Mora-Lopez F, Bernal-Quiros M, Lechuga-Sancho AM, Lechuga-Campoy JL, Hernandez-Trujillo N, Nieto A. Novel mutation in the epithelial sodium channel causing type I pseudohypoaldosteronism in a patient misdiagnosed with cystic fibrosis. Eur J Pediatr. 2012 Jun;171(6):997-1000. doi: 10.1007/s00431-012-1697-5. Epub 2012 Feb 28. PubMed PMID: 22371258.
57. Riepe FG, van Bemmelen MX, Cachat F, Plendl H, Gautschi I, Krone N, Holterhus PM, Theintz G, Schild L. Revealing a subclinical salt-losing phenotype in carriers of the novel S562P mutation in the alpha subunit of the epithelial sodium channel. Clin Endocrinol (Oxf). 2009 Feb;70(2):252-8. doi: 10.1111/j.1365-2265.2008.03314.x. PubMed PMID: 18547339.
58. Riepe FG. Clinical and molecular features of type 1 pseudohypoaldosteronism. Horm Res. 2009;72(1):1-9. doi: 10.1159/000224334. Epub 2009 Jun 30. Review. PubMed PMID: 19571553.
59. Rossier BC, Canessa CM, Schild L, Horisberger JD. Epithelial sodium channels. Curr Opin Nephrol Hypertens. 1994 Sep;3(5):487-96. Review. PubMed PMID: 7804746.
60. Ruffieux-Daidié D, Poirot O, Boulkroun S, Verrey F, Kellenberger S, Staub O. Deubiquitylation regulates activation and proteolytic cleavage of ENaC. J Am Soc Nephrol. 2008 Nov;19(11):2170-80. doi: 10.1681/ASN.2007101130. Epub 2008 Aug 13. PubMed PMID: 18701608; PubMed Central PMCID: PMC2573013.
61. Saravanapandian N, Paul S, Matthai J. Pseudohypoaldosteronism type 1: a rare cause of severe dyselectrolytemia and cardiovascular collapse in neonates. J Clin Neonatol. 2012 Oct;1(4):224-6. doi: 10.4103/2249-4847.106007. PubMed PMID: 24027733; PubMed Central PMCID: PMC3762049.
62. Saxena A, Hanukoglu I, Saxena D, Thompson RJ, Gardiner RM, Hanukoglu A. Novel mutations responsible for autosomal recessive multisystem pseudohypoaldosteronism and sequence variants in epithelial sodium channel alpha-, beta-, and gamma-subunit genes. J Clin Endocrinol Metab. 2002 Jul;87(7):3344-50. PubMed PMID: 12107247.
63. Schaedel C, Marthinsen L, Kristoffersson AC, Kornfält R, Nilsson KO, Orlenius B, Holmberg L. Lung symptoms in pseudohypoaldosteronism type 1 are associated with deficiency of the alpha-subunit of the epithelial sodium channel. J Pediatr. 1999 Dec;135(6):739-45. PubMed PMID: 10586178.
64. Shabbir W, Scherbaum-Hazemi P, Tzotzos S, Fischer B, Fischer H, Pietschmann H, Lucas R, Lemmens Gruber R. Mechanism of action of novel lung edema therapeutic AP301 by activation of the epithelial sodium channel. Mol Pharmacol. 2013 Dec;84(6):899-910. doi: 10.1124/mol. 113.089409. Epub 2013 Sep 27. PubMed PMID: 24077967; PubMed Central PMCID: PMC3834145.
65. Sharma R, Pandey M, Kanwal SK, Zennaro MC. Pseudohypoaldosteronism type 1: management issues. Indian Pediatr. 2013 Mar;50(3):331-3. PubMed PMID: 23680607.
66. Silva N, Costa M, Silva A, Sá C, Martins S, Antunes A, Marques O, Castedo S, Pereira A. A case of systemic pseudohypoaldosteronism with a novel mutation in the SCNN1A gene. Endocrinol Nutr. 2013 Jan;60(1):33-6. doi: 10.1016/j.endonu.2012.07.002. Epub 2012 Sep 30. PubMed PMID: 23031435.
67. Staruschenko A. Regulation of transport in the connecting tubule and cortical collecting duct. Compr Physiol. 2012 Apr;2(2):1541-84. Review. PubMed PMID: 23227301; PubMed Central PMCID: PMC3516049.
68. Stockand JD, Staruschenko A, Pochynyuk O, Booth RE, Silverthorn DU. Insight toward epithelial Na+ channel mechanism revealed by the acid-sensing ion channel 1 structure. IUBMB Life. 2008 Sep;60(9):620-8. doi: 10.1002/iub.89. Review. PMID: 18459164.
69. Strautnieks SS, Thompson RJ, Gardiner RM, Chung E. A novel splice-site mutation in the gamma subunit of the epithelial sodium channel gene in three pseudohypoaldosteronism type 1 families. Nat Genet. 1996 Jun;13(2):248-50. PubMed PMID: 8640238.
70. Strautnieks SS, Thompson RJ, Hanukoglu A, Dillon MJ, Hanukoglu I, Kuhnle U, Seckl J, Gardiner RM, Chung E. Localisation of pseudohypoaldosteronism genes to chromosome 16p12.2-13.11 and 12p13.1-pter by homozygosity mapping. Hum Mol Genet. 1996 Feb;5(2):293-9. PubMed PMID: 8824886.
71. Thomas CP, Zhou J, Liu KZ, Mick VE, MacLaughlin E, Knowles M. Systemic pseudohypoaldosteronism from deletion of the promoter region of the human Beta epithelial na(+) channel subunit. Am J Respir Cell Mol Biol. 2002 Sep;27(3):314-9. PubMed PMID: 12204893.
72. Tzotzos S, Fischer B, Fischer H, Pietschmann H, Lucas R, Dupré G, Lemmens-Gruber R, Hazemi P, Prymaka V, Shabbir W. AP301, a synthetic peptide mimicking the lectin-like domain of TNF, enhances amiloride-sensitive Na(+) current in primary dog, pig and rat alveolar type II cells. Pulm Pharmacol Ther. 2013 Jun;26(3):356-63. doi:10.1016/j.pupt.2012.12.011. Epub 2013 Jan 9. PubMed PMID: 23313096; PubMed Central PMCID: PMC3646188.
73. Vadász I, Schermuly RT, Ghofrani HA, Rummel S, Wehner S, Mühldorfer I, Schäfer KP, Seeger W, Morty RE, Grimminger F, Weissmann N. The lectin-like domain of tumor necrosis factor alpha improves alveolar fluid balance in injured isolated rabbit lungs. Crit Care Med. 2008 May;36(5):1543 50. PubMed PMID: 18434905.
74. Waldmann R, Champigny G, Bassilana F, Voilley N, Lazdunski M. Molecular cloning and functional expression of a novel amiloride-sensitive Na+ channel. J Biol Chem. 1995 Nov 17;270(46):27411-4. PubMed PMID: 7499195.
75. Wang J, Yu T, Yin L, Li J, Yu L, Shen Y, Yu Y, Shen Y, Fu Q. Novel mutations in the SCNN1A gene causing Pseudohypoaldosteronism type 1. PLoS One. 2013 Jun 6;8(6):e65676. doi: 10.1371/journal.pone.0065676. Print 2013. PubMed PMID: 23762408; PubMed Central PMCID: PMC3675083.
76. Welzel M, Akin L, Büscher A, Güran T, Hauffa BP, Högler W, Leonards J, Karges B, Kentrup H, Kirel B, Senses EE, Tekin N, Holterhus PM, Riepe FG. Five novel mutations in the SCNN1A gene causing autosomal recessive pseudohypoaldosteronism type 1. Eur J Endocrinol. 2013 Apr 15;168(5):707-15. doi: 10.1530/EJE-12-1000. Print 2013 May. PubMed PMID: 23416952.
77. Wong GP, Levine D. Congenital pseudohypoaldosteronism presenting in utero with acute polyhydramnios. J Matern Fetal Med. 1998 Mar-Apr;7(2):76-8. PubMed PMID: 9584819.
78. Zennaro MC, Lombès M. Mineralocorticoid resistance. Trends Endocrinol Metab. 2004 Aug;15(6):264-70. Review. PubMed PMID: 15358279.

## Claims

1. A cyclic polypeptide comprising at least six contiguous amino acids from the amino acid sequence SEQ ID NO:1
Gly-Gln-Arg-Glu-Thr-Pro-Glu-Gly-Ala-Glu-Ala-Lys-Pro-Trp-Tyr for the use in the treatment of autosomal recessive pseudohypoaldosteronism type 1B (PHA type1B).

2. Cyclic polypeptide for use according to claim 1, **characterized in that** the cyclic polypeptides comprises the amino acid sequence SEQ ID NO:5
Thr-Pro-Glu-Gly-Ala-Glu
of SEQ ID NO:1.

3. Cyclic polypeptide for use according to claim 1, **characterized by** at least seven contiguous amino acids from the amino acid sequence SEQ ID NO:1.

4. Cyclic polypeptide for use according to one of claims 1 to 3, **characterized by** the amino acid sequence SEQ ID NO:1 Gly-Gln-Arg-Glu-Thr-Pro-Glu-Gly-Ala-Glu-Ala-Lys-Pro-Trp-Tyr.

5. Cyclic polypeptide for use according to one of claims 1 to 4, **characterized in that** the cyclic ring includes a disulfide bond between two cysteine amino acids.

6. Cyclic polypeptide for use according one of claims 1 to 5, **characterized in that** the polypeptide comprises SEQ ID NO:2 Cys-Gly-Gln-Arg-Glu-Thr-Pro-Glu-Gly-Ala-Glu-Ala-Lys-Pro-Trp-Tyr-Cys.

7. Cyclic polypeptide for use according to one of claims 1 to 4, **characterized in that** the cyclic ring includes a non-natural amino acid.

8. Cyclic polypeptide for use according claim 7, **characterized in that** the non-natural amino acid is γ-aminobutyric acid (GABA).

9. Cyclic polypeptide for use according to one of claims 1 to 8, **characterized in that** the cyclic ring comprises at least 10, preferably at least 14 amino acids of SEQ ID NO:1.

10. Cyclic polypeptide for use according to one of claims 1 to 9, **characterized in that** it is or

11. A pharmaceutical composition for the use in the treatment of autosomal recessive pseudohypoaldosteronism type 1B (PHA type1B) comprising at least one cyclic polypeptide as defined in one of claims 1 to 10.

12. A pharmaceutical composition for use according to claim 11, **characterized by** at least one pharmaceutical carrier molecule.

## Patentansprüche

1. Zyklisches Polypeptid, umfassend mindestens sechs zusammenhängende Aminosäuren aus der Aminosäuresequenz SEQ ID Nr: 1 Gly-Gln-Arg-Glu-Thr-Pro-Glu-Gly-Ala-Glu-Ala-Lys-Pro-Trp-Tyr zur Verwendung bei der Behandlung von autosomalem rezessiven Pseudohypoaldosteronismus Type 1 B (PHA Type 1B).

2. Zyklisches Polypeptid zur Verwendung gemäß Anspruch 1, **dadurch gekennzeichnet, dass** das zyklische Polypeptid die Aminosäuresequenz SEQ ID Nr:5 Thr-Pro-Glu-Gly-Ala-Glu
von SEQ ID Nr:1 umfasst.

3. Zyklisches Polypeptid zur Verwendung gemäß Anspruch 1, **gekennzeichnet durch** mindestens sieben zusammenhängende Aminosäuren aus der Aminosäuresequenz SEQ ID Nr:1.

4. Zyklisches Polypeptid zur Verwendung gemäß einem der Ansprüche 1 bis 3, **gekennzeichnet durch** die Aminosäuresequenz SEQ ID Nr: 1 Gly-Gln-Arg-Glu-Thr-Pro-Glu-Gly-Ala-Glu-Ala-Lys-Pro-Trp-Tyr.

5. Zyklisches Polypeptid zur Verwendung gemäß einem der Ansprüche 1 bis 4, **dadurch gekennzeichnet, dass** der zyklische Ring eine Disulfidbindung zwischen zwei Cystein-Aminosäuren umfasst.

6. Zyklisches Polypeptid zur Verwendung gemäß einem der Ansprüche 1 bis 5, **dadurch gekennzeichnet, dass** das Polypeptid die SEQ ID Nr:2 Cys-Gly-Gln-Arg-Glu-Thr-Pro-Glu-Gly-Ala-Glu-Ala-Lys-Pro-Trp-Tyr-Cys umfasst.

7. Zyklisches Polypeptid zur Verwendung gemäß einem der Ansprüche 1 bis 4, **dadurch gekennzeichnet, dass** der zyklische Ring eine nicht-natürliche Aminosäure umfasst.

8. Zyklisches Polypeptid zur Verwendung gemäß Anspruch 7, **dadurch gekennzeichnet, dass** die nicht-natürliche Aminosäure γ-Aminobuttersäure (GABA) ist.

9. Zyklisches Polypeptid zur Verwendung gemäß einem der Ansprüche 1 bis 8, **dadurch gekennzeichnet, dass** der zyklische Ring mindestens 10, vorzugsweise mindestens 14, Aminosäuren von SEQ ID Nr: 1 umfasst.

10. Zyklisches Polypeptid zur Verwendung gemäß einem der Ansprüche 1 bis 9, **dadurch gekennzeichnet, dass** es oder

11. Pharmazeutische Zusammensetzung zur Verwendung bei der Behandlung von autosomalem rezessiven Pseudohypoaldosteronismus Type 1 B (PHA Type 1B), die mindestens ein zyklisches Polypeptid umfasst, wie in einem der Ansprüche 1 bis 10 definiert.

12. Pharmazeutische Zusammensetzung zur Verwendung gemäß Anspruch 11, **gekennzeichnet durch** mindestens ein pharmazeutisches Trägermolekül.

## Revendications

1. Polypeptide cyclique comprenant au moins six acides aminés contigus de la séquence d'acides aminés SEQ ID NO : 1 Gly-Gln-Arg-Glu-Thr-Pro-Glu-Gly-Ala-Glu-Ala-Lys-Pro-Trp-Tyr pour l'utilisation dans le traitement du pseudohypoaldostéronisme autosomique récessif de type 1B (PHA type 1B).

2. Polypeptide cyclique à utiliser selon la revendication 1, **caractérisé en ce que** les polypeptides cycliques comprennent la séquence d'acides aminés SEQ ID NO : 5 Thr-Pro-Glu-Gly-Ala-Glu
de SEQ ID NO : 1.

3. Polypeptide cyclique à utiliser selon la revendication 1, **caractérisé par** au moins sept acides aminés contigus de la séquence d'acides aminés SEQ ID NO : 1.

4. Polypeptide cyclique à utiliser selon l'une des revendications 1 à 3, **caractérisé par** la séquence d'acides aminés SEQ ID NO : 1 Gly-Gln-Arg-Glu-Thr-Pro-Glu-Gly-Ala-Glu-Ala-Lys-Pro-Trp-Tyr.

5. Polypeptide cyclique à utiliser selon l'une des revendications 1 à 4, **caractérisé en ce que** le cycle cyclique comprend une liaison disulfure entre deux acides aminés de cystéine.

6. Polypeptide cyclique à utiliser selon l'une des revendications 1 à 5, **caractérisé en ce que** le polypeptide comprend SEQ ID NO : 2 Cys-Gly-Gln-Arg-Glu-Thr-Pro-Glu-Gly-Ala-Glu-Ala-Lys-Pro-Trp-Tyr-Cys.

7. Polypeptide cyclique à utiliser selon l'une des revendications 1 à 4, **caractérisé en ce que** le cycle cyclique comprend un acide aminé non naturel.

8. Polypeptide cyclique à utiliser selon la revendication 7, **caractérisé en ce que** l'acide aminé non naturel est l'acide y-aminobutyrique (GABA).

9. Polypeptide cyclique à utiliser selon l'une des revendications 1 à 8, **caractérisé en ce que** le cycle cyclique comprend au moins 10, de préférence au moins 14 acides aminés de SEQ ID NO : 1.

10. Polypeptide cyclique à utiliser selon l'une des revendications 1 à 9, **caractérisé en ce qu'**il est SEQ ID NO : 3

11. Composition pharmaceutique destinée à être utilisée dans le traitement du pseudohypoaldostéronisme autosomique récessif de type 1B (type PHA 1B) comprenant au moins un polypeptide cyclique tel que défini dans l'une des revendications 1 à 10.

12. Composition pharmaceutique à utiliser selon la revendication 11, **caractérisée par** au moins une molécule de support pharmaceutique.


### SEQUENCE LISTING

<110> APEPTICO Forschung und Entwicklung GmbH
<120> Cyclic polypeptide for the treatment of PHA type1B
<130> TY10471
<160> 5
<170> PatentIn version 3.5
<210> 1
   <211> 15
   <212> PRT
   <213> Artificial Sequence
<220>
   <223> SEQ ID NO:1
<400> 1
   Gly Gln Arg Glu Thr Pro Glu Gly Ala Glu Ala Lys Pro Trp Tyr
   1               5                   10                  15
<210> 2
   <211> 17
   <212> PRT
   <213> Artificial Sequence
<220>
   <223> SED ID NO:2
<400> 2
   Cys Gly Gln Arg Glu Thr Pro Glu Gly Ala Glu Ala Lys Pro Trp Tyr
   1               5                   10                  15
   Cys
<210> 3
   <211> 17
   <212> PRT
   <213> Artificial Sequence
<220>
   <223> Ring closure between the C-terminal Cys and N-terminal Cys via disulfide bond
<400> 3
   Cys Gly Gln Arg Glu Thr Pro Glu Gly Ala Glu Ala Lys Pro Trp Tyr
   1               5                   10                  15
   Cys
<210> 4
   <211> 16
   <212> PRT
   <213> Artificial Sequence
<220>
   <223> Ring closure between C-term. Asp and N-term. Gly via amide bond between the amino grp. of N-term. Gly and c1-carboxyl group of gamma-aminobutyric acid and between the amino grp. of the Gamma-aminobutyric acid and C of the carboxyl grp. of the
<400> 4
   Gly Gln Arg Glu Thr Pro Glu Gly Ala Glu Ala Lys Pro Trp Tyr Asp
   1               5                   10                  15
<210> 5
   <211> 6
   <212> PRT
   <213> Artificial Sequence
<220>
   <223> SEQ ID NO:5
<400> 5
   Thr Pro Glu Gly Ala Glu
   1               5